(19) Europäisches Patentamt
European Patent Office
Office européen des brevets

(11) **EP 4 328 294 A1**

(12) **EUROPEAN PATENT APPLICATION**
published in accordance with Art. 153(4) EPC

(43) Date of publication:
**28.02.2024 Bulletin 2024/09**

(21) Application number: **22876840.4**

(22) Date of filing: **28.09.2022**

(51) International Patent Classification (IPC):
*C12M 1/12* [(2006.01)]    *C12N 11/14* [(2006.01)]
*C12N 11/06* [(2006.01)]    *C12N 11/08* [(2020.01)]

(52) Cooperative Patent Classification (CPC):
**C12M 1/12; C12N 11/06; C12N 11/08; C12N 11/14**

(86) International application number:
**PCT/KR2022/014551**

(87) International publication number:
**WO 2023/055068 (06.04.2023 Gazette 2023/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.09.2021 KR 20210128333**
**06.09.2022 KR 20220112921**

(71) Applicant: **LG Chem, Ltd.**
**Yeongdeungpo-gu**
**Seoul 07336 (KR)**

(72) Inventors:
• **KIM, Minchae**
**Daejeon 34122 (KR)**
• **KIM, Jee Seon**
**Daejeon 34122 (KR)**
• **KIM, Yunseop**
**Daejeon 34122 (KR)**

(74) Representative: **Plasseraud IP**
**66, rue de la Chaussée d'Antin**
**75440 Paris Cedex 09 (FR)**

(54) **MICROCARRIER FOR CELL CULTURE, METHOD FOR PRODUCING SAME, AND CELL CULTURE COMPOSITION USING SAME**

(57)    The present invention relates to a microcarrier for cell culture comprising: polystyrene-based particles that includes at least one of a hydrocarbon oil having 12 or more carbon atoms, or a void derived therefrom; and magnetic particles.

【FIG. 1】

EP 4 328 294 A1

**Description**

**[TECHNICAL FIELD]**

CROSS-REFERENCE TO RELATED APPLICATION(S)

This application claims the benefit of Korean Patent Application No. 10-2021-0128333 filed on September 28, 2021 and Korean Patent Application No. 10-2022-0112921 filed on September 6, 2022 in the Korean Intellectual Property Office, the entire contents of which are incorporated herein by reference.

The present invention relates to a microcarrier for cell culture, a method for producing the same, and a cell culture composition using the same.

**[BACKGROUND]**

As the biopharmaceutical and regenerative medical fields expand, there is a growing demand for large-scale cell culture technology capable of efficiently producing cells, tissues, microorganisms, and the like.

Adherent cells are cultured using micro carriers in a 3D bioreactor. The cells, culture medium and microcarrier are placed in the bioreactor, and the culture medium is agitated to bring the cells into contact with the micro carriers, so that the cells are adhered onto the surface of the micro carrier and cultured. Since the micro carrier used at this time provides a high surface area/volume ratio to which cells can attach and grow, it is suitable for large-scale culture of cells.

Currently commercially available microcarriers have a density of about 1.1 to 1.3 $g/cm^3$, and cells have a density of about 1.2 $g/cm^3$. In this case, it is advantageous for attaching cells at the initial stage of culture in the bioreactor, but it is difficult to perform centrifugation when separating and recovering cells after culture, and a filtering method based on the microcarrier and cell size should be used. However, in this case, there are problems that the filter is clogged or the process requires a long time, and physical damage and contamination of cells may easily occur, and loss of cells may occur.

Therefore, there is a need to develop a new microcarrier that has a low density so as to enable cell culture, and also can be more easily separated when separating and recovering microcarriers and cells after cell culture.

**[BRIEF SUMMARY]**

**[Technical Problem]**

It is an object of the present invention to provide a microcarrier for cell culture that has a low density so as to enable cell culture, and also can be more easily separated when separating and recovering microcarriers and cells after cell culture.

It is another object of the present invention to provide a method for producing the microcarrier for cell culture.

It is yet another object of the present invention to provide a cell culture composition using the microcarrier for cell culture.

**[Technical Solution]**

In order to achieve the above object, provided herein is a microcarrier for cell culture comprising: polystyrene-based particles that includes at least one of a hydrocarbon oil having 12 or more carbon atoms, or a void derived therefrom; and magnetic particles.

Also provided herein is a method for producing a microcarrier for cell culture, which comprises a step of subjecting a monomer composition containing magnetic particles and a styrene monomer to a suspension polymerization reaction in the presence of a hydrocarbon oil having 12 or more carbon atoms.

Further provided herein is a cell culture composition comprising a cell and the microcarrier for cell culture.

A microcarrier for cell culture, a method for producing the same, and a cell culture composition using the same according to specific embodiments of the present invention will be described in more detail below.

Unless explicitly stated herein, the technical terms used herein are for the purpose of describing specific embodiments only and is not intended to limit the scope of the invention.

The singular forms "a," "an" and "the" used herein are intended to include plural forms, unless the context clearly indicates otherwise.

It should be understood that the terms "comprise," "include", "have", etc. are used herein to specify the presence of stated feature, region, integer, step, action, element and/or component, but do not preclude the presence or addition of one or more other feature, region, integer, step, action, element, component and/or group.

Further, the terms including ordinal numbers such as "a first", "a second", etc. are used only for the purpose of distinguishing one component from another component, and are not limited by the ordinal numbers. For instance, a first component may be referred to as a second component, or similarly, the second component may be referred to as the first component, without departing from the scope of the present invention.

**[DETAILED DESCRIPTION]**

Now, the present invention will be described in more detail.

According to one embodiment of the invention, there can be provided a microcarrier for cell culture comprising: polystyrene-based particles that includes at least one of a hydrocarbon oil having 12 or more carbon atoms, or a void derived therefrom; and magnetic particles.

The present inventors have found through experiments that in the case of a microcarrier for cell culture, as at least one of a hydrocarbon oil having 12 or more carbon atoms, or a void derived therefrom is included inside polystyrene-based particles, not only the density of microcarriers for cell culture can be lowered, but also a homogeneous spherical microcarrier can be secured at a high yield, and completed the present invention.

The inventors have also found through experiments that as the microcarrier for cell culture includes magnetic particles, the separation and purification can be performed more easily using magnetism when separating and recovering the microcarrier and cells after cell culture, and completed the present invention.

Specifically, the microcarrier for cell culture may include polystyrene-based particles that includes at least one of a hydrocarbon oil having 12 or more carbon atoms, or a void derived therefrom; and magnetic particles. That is, the polystyrene-based particles may include one type of hydrocarbon oil having 12 or more carbon atoms, one type of pore derived from hydrocarbon oil having 12 or more carbon atoms, or a mixture of these two types.

When the polystyrene-based particles include one type of void derived from hydrocarbon oil having 12 or more carbon atoms, or include both the hydrocarbon oil having 12 or more carbon atoms and the void derived from hydrocarbon oil having 12 or more carbon atoms, the polystyrene-based particles may correspond to porous polystyrene-based particles.

In the same manner as in the production method of the polystyrene-based particles of other embodiment described later, a suspension polymerization is performed in the presence of hydrocarbon oil to synthesize the polystyrene-based particles, so that the hydrocarbon oil continues to remain trapped inside the polystyrene particles, or part or all of the trapped hydrocarbon oil may escape under the conditions of high-speed suspension polymerization and stirring to form voids inside the polystyrene particles.

The void refers to an empty space inside the polystyrene-based particles, and may be used to mean a pore, a hollow, a hole, a void, and the like.

The void may be derived from a hydrocarbon oil having 12 or more carbon atoms. Specifically, the void corresponds to a space formed while the hydrocarbon oil having 12 or more carbon atoms is phase-separated from polystyrene during suspension polymerization.

Therefore, "the polystyrene-based particles include only one type of hydrocarbon oil having 12 or more carbon atoms" means that the hydrocarbon oil has been completely phase-separated during the polymerization process, but does not disappear to the outside of the particles during the polymerization and washing process, and "the polystyrene-based particles include only one type of pore derived from hydrocarbon oil having 12 or more carbon atoms" means that the hydrocarbon oil has been completely phase-separated during the polymerization process, but disappears to the outside of the particles during polymerization and washing processes. In addition, "the polystyrene-based particles include both the hydrocarbon oil having 12 or more carbon atoms and the void derived from the hydrocarbon oil having 12 or more carbon atoms" means that the hydrocarbon oil is partially phase-separated and disappeared during the polymerization process and a part thereof remains.

Specifically, the diameter of the void may be 0.1 $\mu$m or more and 5 $\mu$m or less. More specifically, the diameter of the void may be 0.1 $\mu$m or more, 0.5 $\mu$m or more, 1 $\mu$m or more, and 5$\mu$m or less, 4$\mu$m or less, 3$\mu$m or less, and specifically, it may be 0.1 $\mu$m or more 5$\mu$m or less, 0.5 $\mu$m or more 5 $\mu$m or less, 1 $\mu$m or more 5 $\mu$m or less, 0.1 $\mu$m or more 4 $\mu$m or less, 0.5 $\mu$m or more 4 $\mu$m or less, 1 $\mu$m or more 4 $\mu$m or less, 0.1 $\mu$m or more 3 $\mu$m or less, 0.5 $\mu$m or more 3 $\mu$m or less, or 1 $\mu$m or more 3$\mu$m or less.

The method for measuring the diameter of the void is not particularly limited, but for example, after a microcarrier for cell culture is embedded in epoxy, a cross section is prepared through ion milling, and then the shape of the particle cross section can be confirmed with an SEM to measure the diameter of the void inside the particles.

As described above, as the void is derived from the hydrocarbon oil having 12 or more carbon atoms, the diameter of the void can realize small-sized pores of 0.1 $\mu$m or more and 5 $\mu$m or less, which makes it possible to easily adjust the density of microcarriers.

Conventionally, in order to reduce the density of polystyrene particles, foamed styrene has been produced by adding a low boiling point foaming agent during the polystyrene polymerization process and then performing an additional foaming process, but in such a case, large voids having a size of several tens of $\mu$m are formed, which makes it difficult to adjust the density of microcarriers.

The carbon number of the hydrocarbon oil may be 12 or more, or 12 or more and 50 or less, or 12 or more and 16 or less. When the carbon number of the hydrocarbon oil is excessively reduced to less than 12, there is a limitation in that the phase separation rate between hydrocarbon oil and polystyrene during suspension polymerization is lowered, so that the particle surface is not uniform, and a large number of concave-shaped particles are produced.

Specifically, the hydrocarbon oil may include a linear or branched saturated hydrocarbon compound having 12 or more and 50 or less carbon atoms. The linear or branched saturated hydrocarbon compound having 12 or more carbon atoms and 50 or less carbon atoms may be used alone or in combination. Examples of the linear or branched saturated hydrocarbon compound having 12 or more and 50 or less carbon atoms include a normal alkane having 12 to 16 carbon atoms, an isoalkane having 12 to 16 carbon atoms, or mixtures thereof.

More specifically, as the hydrocarbon oil, dodecane having 12 carbon atoms, hexadecane having 16 carbon atoms, or Isopar M (a mixture of isoalkane having 12 or more and 14 or less carbon atoms and isoalkane having 13 or more and 16 or less carbon atoms) can be used.

The hydrocarbon oil may be contained in an amount of 30% by weight or less, or 10% by weight or more and 30% by weight or less, with respect to the total weight (100% by weight) of the dispersive phase composition including a styrene monomer. Specifically, the lower limit of the content of the hydrocarbon oil may be 10% by weight or more, or 11% by weight or more, or 12% by weight or more, or 13% by weight or more, or 14% by weight or more, and the upper limit thereof may be, for example, 30% by weight or less, or 25% by weight or less, or 21% by weight or less.

More specifically, the hydrocarbon oil may be contained in an amount of 10% by weight or more and 30% by weight or less, 11% by weight or more and 30% by weight or less, 12% by weight or more and 30% by weight or less, 13% by weight or more and 30% by weight or less, 14% by weight or more and 30% by weight or less, 10% by weight or more and 25% by weight or less, 11% by weight or more and 25% by weight or less, 12% by weight or more and 25% by weight or less, 13% by weight or more and 25% by weight or less, 14% by weight or more and 25% by weight or less, 10% by weight or more and 20% by weight or less, 11% by weight or more and 20% by weight or less, 12% by weight or more and 20% by weight or less, 13% by weight or more and 20% by weight or less, or 14% by weight or more and 20% by weight or less, with respect to the total weight (100% by weight) of the dispersive phase composition including a styrene monomer.

When the content of the hydrocarbon oil is used below the content range, it is difficult to secure carrier particles with low density characteristics, and when the content range is excessively exceeded, the shape of the polystyrene-based particles is hard to have a spherical shape, and the uniformity of the produced particles is reduced.

The density of the hydrocarbon oil may be 0.75 g/cm$^3$ or more and 0.80 g/cm$^3$ or less, 0.75 g/cm$^3$ or more and 0.795 g/cm$^3$ or less, or 0.75 g/cm$^3$ or more and 0.791 g/cm$^3$ or less. Since the density of the hydrocarbon oil is very low in the above-mentioned range, the density of the polystyrene-based particles can be significantly reduced accordingly.

However, when the density of the hydrocarbon oil is excessively reduced to less than 0.75 g/cm$^3$, there is a limitation in that the phase separation rate between hydrocarbon oil and polystyrene during suspension polymerization is lowered, so that the particle surface is not uniform, and a large number of concave-shaped particles are produced.

The apparent density of the microcarrier may be 0.95 g/cm$^3$ or more and less than 1.05 g/cm$^3$. Specifically, the apparent density of the microcarrier may be 0.95 g/cm$^3$ or more, 0.99 g/cm$^3$ or more, 0.995 g/cm$^3$ or more, 0.996 g/cm$^3$ or more, and less than 1.05 g/cm$^3$, 1.049 g/cm$^3$ or less, 1.04 g/cm$^3$ or less, or 1.03 g/cm$^3$ or less, and specifically, it may be 0.95 g/cm$^3$ or more and less than 1.05 g/cm$^3$, 0.99 g/cm$^3$ or more and less than 1.05 g/cm$^3$, 0.995 g/cm$^3$ or more and less than 1.05 g/cm$^3$, 0.996 g/cm$^3$ or more and less than 1.05 g/cm$^3$, 0.95 g/cm$^3$ or more and 1.049 g/cm$^3$ or less, 0.99 g/cm$^3$ or more and 1.049 g/cm$^3$ or less, 0.995 g/cm$^3$ or more and 1.049 g/cm$^3$ or less, 0.996 g/cm$^3$ or more and 1.049 g/cm$^3$ or less, 0.95 g/cm$^3$ or more and 1.04 g/cm$^3$ or less, 0.99 g/cm$^3$ or more and 1.04 g/cm$^3$ or less, 0.995 g/cm$^3$ or more and 1.04 g/cm$^3$ or less , 0.996 g/cm$^3$ or more and 1.04 g/cm$^3$ or less, 0.95 g/cm$^3$ or more and 1.03 g/cm$^3$ or less, 0.99 g/cm$^3$ or more and 1.03 g/cm$^3$ or less , 0.995 g/cm$^3$ or more and 1.03 g/cm$^3$ or less, or 0.996 g/cm$^3$ or more 1.03 g/cm$^3$ or less.

As the microcarrier has the above-mentioned low density range, the cells and microcarriers can be easily separated through the difference in settling velocity due to gravity when separating and recovering microcarriers and cells after cell culture.

The method for measuring the apparent density of the microcarrier is not particularly limited, but for example, a microcarrier sample in a condition where the drying process is completed can be added to an ethanol aqueous solution and distilled water whose density has been adjusted, and the apparent density can be measured by confirming whether the particles float or settle.

More specifically, the microcarrier sample in the condition where the drying process has been completed is added to an aqueous ethanol solution having a density of 0.95 g/cm$^3$, 0.97 g/cm$^3$, 0.98 g/cm$^3$ or 0.995 g/cm$^3$, and distilled water (DIW) having a density of 1 g/cm$^3$, respectively, under the conditions of room temperature (25°C) and atmospheric pressure (1 atm), and the apparent density can be evaluated by confirming whether the particles float or settle.

For example, it can be evaluated that in the case of floating in an aqueous ethanol solution having a density of 0.95 g/cm$^3$, the apparent density of the microcarrier is less than 0.95 g/cm$_3$; in the case of settling in distilled water (DIW) having a density of 1 g/cm$^3$, the apparent density of the microcarrier is greater than 1 g/cm$^3$; in the case of settling in an aqueous ethanol solution having a density of 0.95 g/cm$^3$ and floating in distilled water (DIW) having a density of 1 g/cm$^3$, the apparent density of the microcarrier is 0.95 g/cm$^3$ or more and less than 1 g/cm$^3$; in the case of settling in an aqueous ethanol solution having a density of 0.95 g/cm$^3$ and floating in an aqueous solution of ethanol having a density of 0.97 g/cm$^3$, the apparent density of the microcarrier is 0.95 g/cm3 or more and less than 0.97 g/cm$^3$; and in the case of settling in an aqueous ethanol solution having a density of 0.98 g/cm$^3$ and floating in an aqueous ethanol solution having a density of 0.995 g/cm$^3$, the apparent density of the microcarrier is 0.98 g/cm$^3$ or more and less than 0.995 g/cm$^3$.

When the density of the microcarrier is greater than 1.05 g/cm$^3$, the difference in density between cells and microcarriers is small, and centrifugation may be difficult when separating and recovering cells after culture. When the density of the microcarrier is less than 0.95 g/cm$^3$, the microcarrier floats only on the surface of the culture medium at the initial state of culture, which may cause a problem that it is difficult to attach cells.

A cell to which the microcarrier for cell culture of the embodiment is applied is an adherent animal cell, and examples thereof are not particularly limited, but for example, the cell may be fibroblasts, epithelial cells, osteoblasts, chondrocytes, hepatocytes, human-derived cord blood cells, human bone marrow-derived mesenchymal stem cells, CHO (Chinese hamster ovary) cells, kidney cells (HEK293, BHK21, MDCK, Velocell, etc.), or mixtures of two or more thereof.

The density of the cells may be 1.02 g/cm$^3$ or more and less than 1.1 g/cm$^3$.

Further, the density difference between the cell culture microcarrier and the cells may be 0.02 g/cm$^3$ or more and 0.20 g/cm$^3$ or less. Even if the density difference between the microcarrier for cell culture and the cells is not less than 0.02 g/cm$^3$ and not greater than 0.20 g/cm$^3$ and thus the difference in density is not large, cells and microcarriers can be easily separated by including the magnetic particles.

Meanwhile, the magnetic particles may include magnetic particles that are surface-treated with a hydrophobic functional group. The magnetic particles that are surface-treated with a hydrophobic functional group may refer to magnetic particles in which a hydrophobic functional group is bonded to the surface of the magnetic particles.

Specifically, the surface treatment means that in order to prevent aggregation of magnetic particles and improve dispersibility, the magnetic particles are reacted with a compound containing a hydrophobic functional group to bind the hydrophobic functional group to the surface of the magnetic particles.

In one embodiment, the magnetic particles refers to particles exhibiting magnetic properties. All materials interact with a magnetic field, generating either an attractive force or a repulsive force. That is, if a magnetic field is applied to a material, the material becomes magnetized. The magnetized material is classified into a ferromagnetic material, a paramagnetic material, an antiferromagnetic material, and a ferrimagnetic material depending on the form in which an object is magnetized.

The magnetic particles may be fabricated by solution synthesis, coprecipitation, a sol-gel process, high energy spallation, hydro-thermal synthesis, micro-emulsion synthesis, synthesis by thermal decomposition, or sonic chemical synthesis, but is not limited thereto.

The diameter of the magnetic particles that are surface-treated with a hydrophobic functional group may be 0.1 nm or more and 1000 nm or less. That is, the magnetic particles that are surface-treated with a hydrophobic functional group may be magnetic nanoparticles surface-treated with the hydrophobic functional group.

The nanoparticles refer to particles having a nanometer (nm) size. The nanometer size is a reduction of a micrometer size ($10^{-6}$) to one-thousandth, and if the size of a material is reduced to a nanometer level, it exhibits various and unique physical, chemical, mechanical and electronic properties.

If the size of the magnetic material is reduced to nanometer, the nanoparticles form their respective magnetic regions. In the colloid solution containing these particles, its magnetic dipoles are oriented in irregular directions by thermal fluctuations of the particles, such that the net magnetic force may be "0" superficially. However, when the magnetic force of an externally applied magnetic field is greater than an inner thermal energy, the magnetic dipoles of the particles are aligned in one direction, thereby becoming magnetic materials.

The type of the magnetic particles is not particularly limited, and metal nanoparticles may be used. Examples thereof may include one or more metals or oxides thereof selected from the group consisting of gold (Au), silver (Ag), cobalt (Co), copper (Cu), iron (Fe), chromium (Cr), nickel (Ni), palladium (Pd), platinum (Pt) and tin (Sn). Specifically, the magnetic particles may include iron (Fe) particles.

The magnetic particles may include a hydrophobic ligand that contains a hydrophobic functional group on the surface of the magnetic particles. That is, the magnetic particles that are surface-treated with a hydrophobic functional group may include a hydrophobic ligand that contains a hydrophobic functional group on the surface of the magnetic particles.

As the microcarrier includes magnetic particles that are surface-treated with a hydrophobic functional group, metal particles are independently dispersed to prevent aggregation of particles as well as improve oxidation stability, as compared to microcarriers containing magnetic particles that are not surface-treated.

The ligand refers to a molecule or an ion that is bound around the central metal ion of the compound that is coordinated, and may mean an atom or group of atoms that forms a coordination bond while providing an electron pair to a central metal atom in a complex compound.

That is, the hydrophobic ligand may refer to a hydrophobic molecule or a hydrophobic ion bound around central magnetic particle ions of the magnetic particles that are surface-treated with a hydrophobic functional group.

Specifically, the hydrophobic ligand may include may include one or more ligands selected from the group consisting of a fatty acid having 2 to 20 carbon atoms or a derivative thereof and a fatty acid amine having 2 to 20 carbon atoms or a derivative thereof.

The fatty acid derivative may include a fatty acid ion, and the fatty acid amine derivative may include a fatty acid amine ion.

The fatty acid is a compound containing a hydrocarbon chain and a carboxyl group (-COOH) at its terminal, where the hydrocarbon chain retains its hydrophobicity but can interact with a hydrophilic material through a carboxyl group at its terminal. The fatty acid may be an unsaturated fatty acid or a saturated fatty acid.

The unsaturated fatty acid is a fatty acid having one or more double bonds, wherein -COOH or -COO- at its terminal can form a hydrogen bond, an ionic bond, a covalent bond, etc. with a hydrophilic group exposed on the surface of the magnetic particles and/or an unsaturated coordination site of the magnetic particles.

As the unsaturated fatty acid contains a double bond, bent hydrocarbon chains of unsaturated fatty acids can be oriented on the outside of the magnetic particles to form hydrophobic channels.

That is, the above-mentioned hydrophobic functional group may contain hydrocarbon chains derived from a fatty acid having 2 to 20 carbon atoms or a derivative thereof, or a fatty acid amine having 2 to 20 carbon atoms, or a derivative thereof. More specifically, the hydrophobic functional group may include an alkyl group having 2 to 50 carbon atoms or an alkenyl group having 2 to 50 carbon atoms.

Thereby, the hydrophobic ligand may include a hydrophobic functional group and -COOH or -COO- bonded to its terminal.

The type of the fatty acid having 2 to 20 carbon atoms is not particularly limited, but for example, it may include any one of oleic acid, caproic acid, and stearic acid.

Further, the type of fatty acid amine having 2 to 20 carbon atoms is not particularly limited, but for example, it may include any one of oleyl amine, butyl amine, and octyl amine.

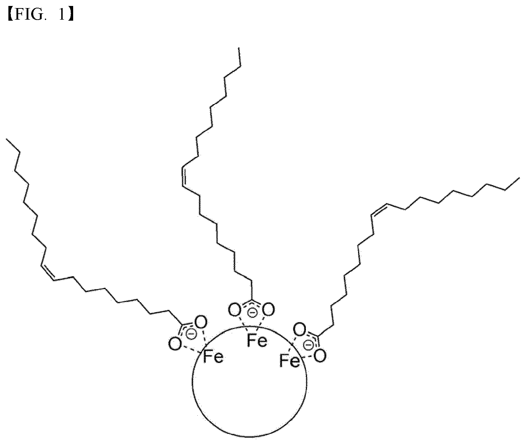

For example, the magnetic particles that are surface-treated with a hydrophobic functional group may have a bonding structure as shown in Fig. 1.

During the production of the magnetic particles that are surface-treated with a hydrophobic functional group, a fatty acid having 2 to 20 carbon atoms is added to a basic aqueous solution to form a fatty acid ion having 2 to 20 carbon atoms, and then reacted with magnetic nanoparticles, whereby a coordination bond between -COO-contained at the terminal of a fatty acid having 2 to 20 carbon atoms and the magnetic nanoparticles may be formed as shown in Fig. 1.

Meanwhile, the density of the magnetic particles may be 5 g/cm$^3$ or more and 6 g/cm$^3$ or less. That is, the density of the magnetic particles that are surface-treated with the hydrophobic functional group may be 5 g/cm$^3$ or more and 6 g/cm$^3$ or less.

Specifically, the density of the magnetic particles may be 5 g/cm$^3$ or more, 5.1 g/cm$^3$ or more, 5.15 g/cm$^3$ or more or 6 g/cm$^3$ or less, 5.8 g/cm$^3$ or less, 5.5 g/cm$^3$ or less, 5.3 g/cm$^3$ or less, or 5 g/cm$^3$ or more and 6 g/cm$^3$ or less, 5 g/cm$^3$ or more and 5.8 g/cm$^3$ or less, 5 g/cm$^3$ or more and 5.5 g/cm$^3$ or less, 5 g/cm$^3$ or more and 5.3g/cm$^3$ or less, 5.1 g/cm$^3$ or more and 6 g/cm$^3$ or less, 5.1 g/cm$^3$ or more and 5.8 g/cm$^3$ or less, 5.1 g/cm$^3$ or more and 5.5 g/cm$^3$ or less, 5.1 g/cm$^3$ or more and 5.3 g/cm$^3$ or less, 5.15 g/cm$^3$ or more and 5.8 g/cm$^3$ or less, 5.15 g/cm$^3$ or more and 5.5 g/cm$^3$ or less, 5.15 g/cm$^3$ or more and 5.3 g/cm$^3$ or less.

As described above, as the density of the magnetic particles is 5 g/cm$^3$ or more and 6 g/cm$^3$ or less, when the magnetic particles are excessively added in an amount of greater than 5 parts by weight with respect to 100 parts by weight of the hydrocarbon oil having 12 or more carbon atoms, the overall density of the particles increases due to excessive inclusion of the high-density magnetic particles, which not only prevents the particles from smoothly floating in an incubator, but can also exhibit cytotoxicity.

Further, the diameter of the magnetic particles that are surface-treated with a hydrophobic functional group may be 100 nm or more and 2000 nm or less. A method of measuring the diameter of the magnetic particles is not particularly limited, but for example, it can be measured using dynamic light scattering (DLS) or the like.

Specifically, the diameter of the magnetic particles that are surface-treated with a hydrophobic functional group may be 100 nm or more, 300 nm or more, 500 nm or more, or 2000 nm or less, 1500 nm or less, 1000 nm or less, 800 nm or less, 600 nm or less, or 100 nm or more and 2000 nm or less, 100 nm or more and 1500 nm or less, 100 nm or more and 1000 nm or less, 100 nm or more and 800 nm or less, 100 nm or more and 600 nm or less, 300 nm or more an 2000 nm or less, 300 nm or more and 1500 nm or less, 300 nm or more and 1000 nm or less, 300 nm or more and 800 nm or less, 300 nm or more and 600 nm or less, or 500 nm or more and 2000 nm or less, 500 nm or more and 1500 nm or less, 500 nm or more and 1000 nm or less, 500 nm or more and 800 nm or less, 500 nm or more and 600 nm or less.

The polystyrene-based particles may include a polystyrene-based polymer in which at least one of a hydrocarbon oil having 12 or more carbon atoms, or a pore derived therefrom; and magnetic particles are dispersed therein.

That is, the polystyrene-based particles may include at least one or more of a polystyrene-based polymer matrix, a hydrocarbon oil having 12 or more carbon atoms dispersed in the polystyrene-based polymer matrix, or a pore derived therefrom; and magnetic particles.

Specifically, the microcarrier for cell culture may exist in a state in which magnetic particles are dispersed inside at least one or more of a hydrocarbon oil having 12 or more carbon atoms, or a pore derived therefrom.

As mentioned above, the microcarrier for cell culture includes a suspension polymerization reaction product of a monomer composition containing a hydrocarbon oil having 12 or more carbon atoms, magnetic particles, and a styrene monomer, and may include pores, which are spaces formed while the hydrocarbon oil having 12 or more carbon atoms is phase-separated from polystyrene during suspension polymerization.

Therefore, as the magnetic particles are included in the monomer composition, these may exist in a state in which magnetic particles are dispersed inside at least one or more of a hydrocarbon oil having 12 or more carbon atoms, or a pore derived therefrom.

Specifically, the polystyrene-based particles may include a suspension polymerization reaction product of a monomer composition containing a hydrocarbon oil having 12 or more carbon atoms, magnetic particles that are surface-treated with a hydrophobic functional group, and a styrene monomer.

The monomer composition may contain 0.01 parts by weight or more and 5 parts by weight or less of magnetic particles with respect to 100 parts by weight of the hydrocarbon oil having 12 or more carbon atoms. Specifically, the monomer composition may contain the magnetic particles in an amount of 0.01 parts by weight or more, 0.02 parts by weight or more, or 0.04 parts by weight or more, and 5 parts by weight or less, 4 parts by weight or less, more specifically, 0.01 parts by weight or more and 5 parts by weight or less, 0.02 parts by weight or more and 5 parts by weight or less, 0.02 parts by weight or more and 4 parts by weight or less, 0.04 parts by weight or more and 4 parts by weight or less, with respect to 100 parts by weight of the hydrocarbon oil having 12 or more carbon atoms.

When the magnetic particles are excessively reduced to less than 0.01 parts by weight with respect to 100 parts by weight of the hydrocarbon oil having 12 or more carbon atoms, there is a limitation in that the magnetism of the microcarrier is not sufficiently realized, and when separating and recovering the microcarrier and the cells after cell culture, separation and purification is not easy, and the recovery process is additionally involved, which makes the process complicate.

Meanwhile, when the magnetic particles are excessively added in an amount of greater than 5 parts by weight with respect to 100 parts by weight of the hydrocarbon oil having 12 or more carbon atoms, the overall density of the particles increases due to excessive inclusion of high-density magnetic particles, which not only prevents the particles from smoothly floating in an incubator but also can exhibit cytotoxicity.

Further, the monomer composition may include the ethylenically unsaturated crosslinking agent in an amount of greater than 0.033 parts by weight and less than 3 parts by weight with respect to 1 part by weight of the styrene monomer.

Specifically, when the amount of the ethylenically unsaturated crosslinking agent is excessively reduced to less than 0.033 parts by weight with respect to 1 part by weight of the styrene monomer, there is a limitation in that the crosslinking density of the polystyrene-based polymer is reduced, the voids generated by the hydrocarbon oil are hard to stably produce, which makes it difficult to sufficiently lower the density of the polystyrene-based particles.

On the other hand, when the amount of the ethylenically unsaturated crosslinking agent is excessively increased to 3 parts by weight or more with respect to 1 part by weight of the styrene monomer, there is a limitation in that while the crosslinking density of the polystyrene-based polymer increases, the overall density of the polystyrene-based particles is hard to decrease to a target level.

Further, the content of the hydrocarbon oil may be 10% by weight or more and 30% by weight or less, or 14% by weight or more and 21% by weight or less with respect to the total weight of the monomer composition. When the content of the hydrocarbon oil is excessively reduced, the amount of hydrocarbon oil impregnated inside the particles is reduced, which makes it difficult to sufficiently lower the density of the polystyrene-based particles. Further, when the content of the hydrocarbon oil is excessively increased, the shape of the polystyrene-based particles is hard to have a spherical shape, and the uniformity of the produced particles is reduced.

Examples of the ethylenically unsaturated crosslinking agent include divinylbenzene.

The ratio of the surface area of the polystyrene-based particles in contact with the micropores existing on the surface of the polystyrene-based particles based on the total surface area of the polystyrene-based particles may be less than 0.01%. The total surface area of the polystyrene-based particles means the sum of the surface areas exposed to the air at the outermost corners of the polystyrene-based particles, and the surface area of the polystyrene-based particles in contact with the micropores existing on the surface of the polystyrene-based particles means the sum of the surface areas in which the micropores existing on the surface of the polystyrene-based particles are in contact with the outermost surface of the polystyrene-based particles. Further, the micropore is a pore having a maximum diameter of a micrometer size, for example, means a pore having a maximum diameter of 1 $\mu$m or more and 500 $\mu$m or less.

More specifically, the ratio of the surface area of the polystyrene-based particles in contact with the micropores existing on the surface of the polystyrene-based particles based on the total surface area of the polystyrene-based particles can be obtained according to the following Equation 1.

[Equation 1]

Ratio of the surface area of the polystyrene-based particles in contact with the micropores existing on the surface of the polystyrene-based particles based on the total surface area of the polystyrene-based particles (%) = [(Surface area of the polystyrene-based particles in contact with the micropores existing on the surface of the polystyrene-based particles) / (Total surface area of the polystyrene-based particles)] * 100

"The ratio of the surface area of the polystyrene-based particles in contact with the micropores existing on the surface of the polystyrene-based particles based on the total surface area of the polystyrene-based particles is less than 0.01%" means that micro-sized pores existing on the surface of the polystyrene-based particles is very small so as to be completely absent or be almost absent. That is, micropores may not exist on the surface of the polystyrene-based particles.

Conventionally, in order to reduce the density of polystyrene particles, foamed styrene has been produced by adding a foaming agent, but in this case, the distribution of the diameter range and the density range of the polystyrene particles became too wide, which makes it difficult to secure a yield within the applicable range as a microcarrier for cell culture.

Meanwhile, the polystyrene-based particles have an advantage in that the distribution of the diameter range and the density range of the polystyrene particles can be more precisely adjusted, because the ratio of the surface area of the polystyrene-based particles in contact with the micropores existing on the surface of the polystyrene - based particles based on the total surface area of the polystyrene-based particles is less than 0.01% and thus, the foaming process by a foaming agent does not proceed at all, unlike the conventional formed styrene.

The microcarrier may have an average diameter of 50 $\mu$m or more and 400 $\mu$m or less.

Specifically, the average diameter of the microcarrier may be 50 $\mu$m or more, 75 $\mu$m or more, 100 $\mu$m or more, 120 $\mu$m or more, or 400 $\mu$m or less, 300 $\mu$m or less, 250 $\mu$m or less, 200 $\mu$m or less, or 50 $\mu$m or more and 400 $\mu$m or less, 50 $\mu$m or more and 300 $\mu$m or less, 50 $\mu$m or more and 250 $\mu$m or less, 50 $\mu$m or more and 200 $\mu$m or less, 50 $\mu$m or more and 400 $\mu$m or less, 75 $\mu$m or more and 300 $\mu$m or less, 75 $\mu$m or more and 250 $\mu$m or less, 75 $\mu$m or more and 200 $\mu$m or less, 100 $\mu$m or more and 400 $\mu$m or less, 100 $\mu$m or more and 300 $\mu$m or less, 100 $\mu$m or more and 250 $\mu$m or less, 100 $\mu$m or more and 200 $\mu$m or less, 120 $\mu$m or more and 400 $\mu$m or less, 120 $\mu$m or more and 300 $\mu$m or less, 50 $\mu$m or more and 120 $\mu$m or less, or 50 $\mu$m or more and 120 $\mu$m or less. When the average diameter of the microcarrier satisfies the above-mentioned range, the cell adhesion and culture performance are excellent. On the other hand, when the average diameter of the microcarrier is less than 50 $\mu$m, there is a risk that the surface area available for cell culture will be small and the culture efficiency will be low, and when the average diameter of the microcarriers is greater than 400 $\mu$m, interactions between adherent cells are reduced, the cell density in the incubator is lowered, which causes a problem that the cell culture efficiency is lowered.

The diameter of the microcarrier means the distance between two points where a straight line passing through the center of gravity of the microcarrier meets the outermost surface of the microcarrier, and the average diameter of the microcarrier can be obtained by confirming the diameter of all particles contained in the microcarrier for cell culture through an optical microscope.

The microcarrier may be a group of individual particles having an average diameter of 50 $\mu$m or more and 400 $\mu$m or less, and individual particles included in such a group may have an average diameter of 50 $\mu$m or more and 400 $\mu$m or less. More specifically, 95%, or 99% of the individual particles included in the group may have a diameter of 50 $\mu$m or more and 400 $\mu$m or less.

Further, the microcarrier may have a specific surface area of 200 cm$^2$/g or more and 1000 cm$^2$/g or less. Specifically, the microcarrier may have a specific surface area of 200 cm$^2$/g or more, 300 cm$^2$/g or more, 330 cm$^2$/g or more, 350 cm$^2$/g or more, and, 1000 cm$^2$/g or less, 500 cm$^2$/g or less, 450 cm$^2$/g or less, more specifically 200 cm$^2$/g or more and 1000 cm$^2$/g or less, 300 cm$^2$/g or more and 1000 cm$^2$/g or less, 300 cm$^2$/g or more and 500 cm$^2$/g or less,

300 cm$^2$/g or more and 450 cm$^2$/g or less, 330 cm$^2$/g or more and 450 cm$^2$/g or less, or 350 cm$^2$/g or more and 450 cm$^2$/g or less.

The method for measuring the specific surface area is not particularly limited, but for example, from the apparent density, the specific surface area of the microcarrier can be calculated using the following Equation.

[Equation]

$$\text{Specific surface area of microcarrier (cm}^2/\text{g)} = 3/(r*\rho)$$

in the above Equation, r means the average radius of microcarrier, and $\rho$ means the apparent density of microcarrier.

The surface of the polystyrene-based particles may further include a primer polymer layer, a cell adhesion-inducing layer, or a combination layer thereof. That is, the surface of the polystyrene-based particles may further include one type of primer polymer layer, one type of cell adhesion-inducing layer, or a mixed layer of one type of primer polymer layer and one type of cell adhesion-inducing layer. In a mixed layer of one type of primer polymer layer and one type of cell adhesion-inducing layer, the stacking order of these is not particularly limited, and both a structure in which a cell adhesion-inducing layer is laminated on a primer polymer layer or a structure in which a primer polymer layer is laminated on a cell adhesion-inducing layer are applicable.

Meanwhile, the primer polymer layer serves as an adhesive layer that can introduce a functional polymer onto the surface of polystyrene-based particles having no functional group, thereby effectively introducing a polymer layer for cell adhesion onto the surface of the microcarrier, and enabling a stable maintenance even during culture.

Examples of the primer polymer layer are not particularly limited, but it may be a catechol derivative capable of inducing water phase adhesion, which may include any one or more selected from the group consisting of L-dihydroxyphenylalanine (L-DOPA), dopamine, polydopamine, norepinephrine, epinephrine, epigallocatechin and derivatives thereof.

Meanwhile, the cell adhesion-inducing layer is composed of cell adhesion materials, which serve to provide sites where the transmembrane proteins of the cell can bind, thereby allowing adherent cells to be stably adhered, spread and cultured.

Examples of the polymer forming the cell adhesion-inducing layer are not particularly limited, but it may include any one or more selected from the group consisting of gelatin, collagen, fibronectin chitosan, poly dopamine, poly L-lysine, vitronectin, peptide containing RGD, acrylic polymer containing RGD, lignin, cationic dextran and derivatives thereof.

In one example, the microcarrier includes a primer polymer layer formed on the surface of the polystyrene-based particles, and thus, can have the effects of being water-dispersed by modifying the surface of the microcarrier to be hydrophilic, introducing a cell adhesion-inducing layer onto the surface of the primer polymer layer, adjusting the floating degree of microcarrier in the culture medium, and stably attaching/culturing cells.

Meanwhile, the ratio between the radius of the polystyrene-based particles and the thickness of the primer polymer layer may be 1:0.00001 to 1:0.01, or 1:0.0001 to 1:0.001.

When the ratio between the radius of the polystyrene-based particles and the thickness of the surface coating layer is less than 1:0.00001, the primer polymer layer is too thin compared to the polystyrene-based particles, so that the effect of modifying the surface of the microcarrier to be hydrophilic is insignificant, and when the ratio exceeds 1:0.01, the polymer layer of the primer becomes thicker than the polystyrene-based particles, so that the degree of adhesion between the cells and the microcarriers during cell culture may be reduced.

According to another embodiment of the invention, there can be provided a method for producing a microcarrier for cell culture, which comprises subjecting a monomer composition containing magnetic particles and a styrene monomer to a suspension polymerization reaction in the presence of a hydrocarbon oil having 12 or more carbon atoms.

The contents of the hydrocarbon oil, the magnetic particles, and the styrene monomer include all those described above in the one embodiment. That is, the magnetic particles may include magnetic particles that are surface-treated with a hydrophobic functional group.

The magnetic particles that are surface-treated with a hydrophobic functional group may refer to magnetic particles in which a hydrophobic functional group is bonded to the surface of the magnetic particles.

Specifically, the surface treatment means that in order to prevent aggregation of magnetic particles and improve dispersibility, the magnetic particles are reacted with a compound containing a hydrophobic functional group to bind the hydrophobic functional group to the surface of the magnetic particles.

In one embodiment, the magnetic particles refers to particl es exhibiting magnetic properties. All materials interact with a magnetic field, generating either an attractive force or a repulsive force. That is, if a magnetic field is applied to a material, the material becomes magnetized. The magnetized material is classified into a ferromagnetic material, a paramagnetic material, an antiferromagnetic material, and a ferrimagnetic material depending on the form in which an object is magnetized.

The magnetic particles may be fabricated by solution synthesis, coprecipitation, a sol-gel process, high energy spallation, hydro-thermal synthesis, micro-emulsion synthesis, synthesis by thermal decomposition, or sonic chemical synthesis, but is not limited thereto.

The diameter of the magnetic particles that are surface-treated with a hydrophobic functional group may be 0.1 nm or more and 1000 nm or less. That is, the magnetic particles that are surface-treated with a hydrophobic functional group may be magnetic nanoparticles surface-treated with the hydrophobic functional group.

The nanoparticles refer to particles having a nanometer (nm) size. The nanometer size is a reduction of a micrometer size ($10^{-6}$) to one-thousandth, and if the size of a material is reduced to a nanometer level, it exhibits various and unique physical, chemical, mechanical and electronic properties.

If the size of the magnetic material is reduced to nanometer, the nanoparticles form their respective magnetic regions. In the colloid solution containing these particles, its magnetic dipoles are oriented in irregular directions by thermal fluctuations of the particles, such that the net magnetic force may be "0" superficially. However, when the magnetic force of an externally applied magnetic field is greater than an inner thermal energy, the magnetic dipoles of the particles are aligned in one direction, thereby becoming magnetic materials.

The type of the magnetic particles is not particularly limited, and metal nanoparticles may be used. Examples thereof may include one or more metals or oxides thereof selected from the group consisting of gold (Au), silver (Ag), cobalt (Co), copper (Cu), iron (Fe), chromium (Cr), nickel (Ni), palladium (Pd), platinum (Pt) and tin (Sn). Specifically, the magnetic particles may include iron (Fe) particles.

The magnetic particles may include a hydrophobic ligand that contains a hydrophobic functional group on the surface of the magnetic particles. That is, the magnetic particles that are surface-treated with a hydrophobic functional group may include a hydrophobic ligand that contains a hydrophobic functional group on the surface of the magnetic particles.

As the method for producing the microcarrier includes magnetic particles that are surface-treated with a hydrophobic functional group, metal particles are independently dispersed to prevent aggregation of particles as well as improve oxidation stability, as compared to microcarriers containing magnetic particles that are not surface-treated.

The ligand refers to a molecule or an ion that is bound around the central metal ion of the compound that is coordinated, and may mean an atom or group of atoms that forms a coordination bond while providing an electron pair to a central metal atom in a complex compound.

That is, the hydrophobic ligand may refer to a hydrophobic molecule or a hydrophobic ion bound around central magnetic particle ions of the magnetic particles that are surface-treated with a hydrophobic functional group.

Specifically, the hydrophobic ligand may include may include one or more ligands selected from the group consisting of a fatty acid having 2 to 20 carbon atoms or a derivative thereof and a fatty acid amine having 2 to 20 carbon atoms or a derivative thereof.

The fatty acid derivative may include a fatty acid ion, and the fatty acid amine derivative may include a fatty acid amine ion.

The fatty acid is a compound containing a hydrocarbon chain and a carboxyl group (-COOH) at its terminal, where the hydrocarbon chain retains its hydrophobicity but can interact with a hydrophilic material through a carboxyl group at its terminal. The fatty acid may be an unsaturated fatty acid or a saturated fatty acid.

The unsaturated fatty acid is a fatty acid having one or more double bonds, wherein -COOH or -COO- at its terminal can form a hydrogen bond, an ionic bond, a covalent bond, etc. with a hydrophilic group exposed on the surface of the magnetic particles and/or an unsaturated coordination site of the magnetic particles.

As the unsaturated fatty acid contains a double bond, bent hydrocarbon chains of unsaturated fatty acids can be oriented on the outside of the magnetic particles to form hydrophobic channels.

That is, the above-mentioned hydrophobic functional group may contain hydrocarbon chains derived from a fatty acid having 2 to 20 carbon atoms or a derivative thereof, or a fatty acid amine having 2 to 20 carbon atoms, or a derivative thereof. More specifically, the hydrophobic functional group may include an alkyl group having 2 to 50 carbon atoms or an alkenyl group having 2 to 50 carbon atoms.

Thereby, the hydrophobic ligand may include a hydrophobic functional group and -COOH or -COO- bonded to its terminal.

The type of the fatty acid having 2 to 20 carbon atoms is not particularly limited, but for example, it may include any one of oleic acid, caproic acid, and stearic acid.

Further, the type of fatty acid amine having 2 to 20 carbon atoms is not particularly limited, but for example, it may include any one of oleyl amine, butyl amine, and octyl amine.

For example, the magnetic particles that are surface-treated with a hydrophobic functional group may have a bonding structure as shown in Fig. 1.

During the production of the magnetic particles that are surface-treated with a hydrophobic functional group, a fatty acid having 2 to 20 carbon atoms is added to a basic aqueous solution to form a fatty acid ion having 2 to 20 carbon atoms, and then reacted with magnetic nanoparticles, whereby a coordination bond between -COO-contained at the terminal of a fatty acid having 2 to 20 carbon atoms and the magnetic nanoparticles may be formed as shown in Fig. 1.

Meanwhile, the density of the magnetic particles may be 5 $g/cm^3$ or more and 6 $g/cm^3$ or less. That is, the density of the magnetic particles that are surface-treated with the hydrophobic functional group may be 5 $g/cm^3$ or more and 6 $g/cm^3$ or less.

Specifically, the density of the magnetic particles may be 5 $g/cm^3$ or more, 5.1 $g/cm^3$ or more, 5.15 $g/cm^3$ or more or 6 $g/cm^3$ or less, 5.8 $g/cm^3$ or less, 5.5 $g/cm^3$ or less, 5.3 $g/cm^3$ or less, or 5 $g/cm^3$ or more 6 $g/cm^3$ or less, 5 $g/cm^3$ or more 5.8 $g/cm^3$ or less, 5.1 $g/cm^3$ or more 5.8 $g/cm^3$ or less, 5.1 $g/cm^3$ or more 5.5 $g/cm^3$ or less, 5.1 $g/cm^3$ or more 5.3 $g/cm^3$ or less, 5.15 $g/cm^3$ or more 5.3 $g/cm^3$ or less.

As described above, as the density of the magnetic particles is 5 $g/cm^3$ or more and 6 $g/cm^3$ or less, when the magnetic particles are excessively added in an amount of greater than 5 parts by weight with respect to 100 parts by weight of the hydrocarbon oil having 12 or more carbon atoms, the overall density of the particles increases due to excessive inclusion of the high-density magnetic particles, which not only prevents the particles from smoothly floating in an incubator, but can also exhibit cytotoxicity.

Further, the diameter of the magnetic particles that are surface-treated with a hydrophobic functional group may be 100 nm or more and 2000 nm or less.

Specifically, the diameter of the magnetic particles that are surface-treated with a hydrophobic functional group may be 100 nm or more, 300 nm or more, 500 nm or more, or 2000 nm or less, 1500 nm or less, 1000 nm or less, 800 nm or less, 600 nm or less, or 100 nm or more and 2000 nm or less, 100 nm or more and 1500 nm or less, 100 nm or more and 1000 nm or less, 100 nm or more and 800 nm or less, 100 nm or more and 600 nm or less, 300 nm or more and 2000 nm or less, 300 nm or more and 1500 nm or less, 300 nm or more and 1000 nm or less, 300 nm or more and 800 nm or less, 300 nm or more and 600 nm or less, or 500 nm or more and 2000 nm or less, 500 nm or more and 1500 nm or less, 500 nm or more and 1000 nm or less, 500 nm or more and 800 nm or less, 500 nm or more and 600 nm or less.

The monomer composition may contain 0.01 parts by weight or more and 5 parts by weight or less of magnetic particles with respect to 100 parts by weight of the hydrocarbon oil having 12 or more carbon atoms. Specifically, the monomer composition may contain the magnetic particles in an amount of 0.01 parts by weight or more, 0.02 parts by weight or more, or 0.04 parts by weight or more, and 5 parts by weight or less, 4 parts by weight or less, more specifically, 0.01 parts by weight or more and 5 parts by weight or less, 0.02 parts by weight or more and 5 parts by weight or less, 0.02 parts by weight or more and 4 parts by weight or less, 0.04 parts by weight or more and 4 parts by weight or less, with respect to 100 parts by weight of the hydrocarbon oil having 12 or more carbon atoms.

When the magnetic particles are excessively reduced to less than 0.01 parts by weight with respect to 100 parts by weight of the hydrocarbon oil having 12 or more carbon atoms, there is a limitation in that the magnetism of the microcarrier is not sufficiently realized, and when separating and recovering the microcarrier and the cells after cell culture, separation and purification is not easy, and the recovery process is additionally involved, which makes the process complicate.

Meanwhile, when the magnetic particles are excessively added in an amount of greater than 5 parts by weight with respect to 100 parts by weight of the hydrocarbon oil having 12 or more carbon atoms, the overall density of the particles increases due to excessive inclusion of high-density magnetic particles, which not only prevents the particles from smoothly floating in an incubator but also can exhibit cytotoxicity.

Further, the monomer composition may include the ethylenically unsaturated crosslinking agent in an amount of greater than 0.033 parts by weight and less than 3 parts by weight with respect to 1 part by weight of the styrene monomer.

Specifically, when the amount of the ethylenically unsaturated crosslinking agent is excessively reduced to less than 0.033 parts by weight with respect to 1 part by weight of the styrene monomer, there is a limitation in that the crosslinking density of the polystyrene-based polymer is reduced, the voids generated by the hydrocarbon oil are hard to stably produce, which makes it difficult to sufficiently lower the density of the polystyrene-based particles.

On the other hand, when the amount of the ethylenically unsaturated crosslinking agent is excessively increased to 3 parts by weight or more with respect to 1 part by weight of the styrene monomer, there is a limitation in that while the crosslinking density of the polystyrene-based polymer increases, the overall density of the polystyrene-based particles is hard to decrease to a target level.

Further, the content of the hydrocarbon oil may be 10% by weight or more and 30% by weight or less, or 14% by weight or more and 21% by weight or less with respect to the total weight of the monomer composition. When the content of the hydrocarbon oil is excessively reduced, the amount of hydrocarbon oil impregnated inside the particles is reduced, which makes it difficult to sufficiently lower the density of the polystyrene-based particles. Further, when the content of the hydrocarbon oil is excessively increased, the shape of the polystyrene-based particles is hard to have a spherical shape, and the uniformity of the produced particles is reduced.

More specifically, the suspension polymerization reaction of the monomer composition may include a step of mixing the monomer composition into an aqueous dispersion and applying a shearing force thereto to homogenize the monomer composition in the aqueous dispersion in the form of droplets; and a step of subjecting the homogenized monomer composition to a suspension polymerization reaction at a stirring speed of 300 rpm or more and 1000 rpm or less.

During the formation of the particle structure of polystyrene and hydrocarbon oil in the step of subjecting the homogenized monomer composition to a suspension polymerization reaction at a stirring speed of 300 rpm or more and 1000 rpm or less, or 400 rpm or more and 800 rpm or less, it is possible to produce the microcarrier for cell culture having a high ratio of spherical particles while forming internal pores by phase separation of polystyrene and hydrocarbon oil, and lowering the density of the microcarrier for cell culture.

In the step of subjecting the homogenized monomer composition to a suspension polymerization reaction at a stirring speed of 300 rpm or more and 1000 rpm or less, or 400 rpm or more and 800 rpm or less, examples of the suspension polymerization conditions are not particularly limited, but for example, the polymerization may be performed at a temperature of 50°C or more and 100°C for 3 hours or more and 18 hours or less.

Meanwhile, the method for producing a microcarrier for cell culture may further include washing and drying the suspension polymerization reaction product, after the step of performing the suspension polymerization reaction.

Specifically, the step of washing the suspension polymerization reaction product may include a step of filtering the suspension polymerization reaction product through a sieve of 10 $\mu$m or more and 100 $\mu$m or less, and then stirring it 5 to 7 times at room temperature in 100% ethanol and/or distilled water at a high temperature of 50°C or more and 80°C or less.

The step of drying the suspension polymerization reaction product includes a step of placing it in a vacuum oven and vacuum-drying at room temperature. However, the drying method is not limited thereto, and any drying method known to be commonly used can be used without particular limitation

Meanwhile, the method for producing a microcarrier for cell culture of the other embodiment may further include a step of applying a primer polymer layer, a cell adhesion-inducing layer, or a combination layer thereof onto the surface of the suspension polymerization reaction product, after the step of performing the suspension polymerization reaction.

The contents of the primer polymer layer and the cell adhesion-inducing layer include all those described above in the one embodiment.

According to yet another embodiment of the invention, there can be provided a cell culture composition comprising a cell and the microcarrier for cell culture of the one embodiment.

The cell is an adherent animal cell, and examples thereof are not particularly limited, but for example, it may be fibroblasts, epithelial cells, osteoblasts, chondrocytes, hepatocytes, human-derived cord blood cells, human bone marrow-derived mesenchymal stem cells, CHO (Chinese hamster ovary) cells, kidney cells (HEK293, BHK21, MDCK, Velocell, etc.), or mixtures of two or more thereof.

The density of the cells may be 1.02 g/cm$^3$ or more and less than 1.1 g/cm$^3$.

Further, the density difference between the cell culture microcarrier and the cells may be 0.02 g/cm$^3$ or more and 0.20 g/cm$^3$ or less. Even if the density difference between the microcarrier for cell culture and the cells is not less than 0.02 g/cm$^3$ and not greater than 0.20 g/cm$^3$ and thus the difference in density is not large, cells and microcarriers can be easily separated by including the magnetic particles.

A cell culture composition may further include a medium solution. The medium solution may contain nutrients close to biological conditions based on body fluids such as plasma and lymph fluid, and various additives to sufficiently satisfy environmental conditions such as nutrients and pH, temperature, and osmotic pressure. Various substances widely known in the cell culture-related technical field can be used without limitation.

As an example, the microcarrier for cell culture of one embodiment has a density lower than that of the medium solution, and thus is injected into the medium solution and floats in the medium solution under stirring conditions. Then, as the number of cells adhering to the surface of the low-density microcarrier increases, the microcarrier to which the cell is attached (hereinafter, referred to as 'microcarrier-cell conjugate') gradually increases in density and gradually sinks in the medium solution.

Therefore, the microcarrier (microcarrier-cell conjugate) to which the cells are attached is treated by adding a cell detachment enzyme and then separated through centrifugation to separate the cells from the microcarrier-cell conjugate, thereby easily securing the cultured cells.

[Advantageous Effects]

According to the present invention, there can be provided a microcarrier for cell culture that has a low density so as to enable cell culture, and also can be more easily separated when separating and recovering microcarriers and cells after cell culture, a method for producing the same, and a cell culturing method using the same.

[BRIEF DESCRIPTION OF THE DRAWINGS]

Fig. 1 shows magnetic particles that are surface-treated with hydrophobic functional groups.
Fig. 2 shows an SEM image of the microcarrier for cell culture obtained in Example 1.
Fig. 3 shows an SEM image of a particle cross-sectional shape of a microcarrier for cell culture obtained in Example 1.

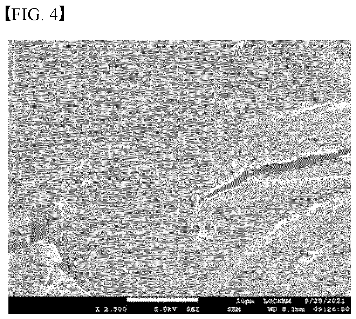

Fig. 4 shows an SEM image of the particle surface shape of the microcarrier for cell culture obtained in Example 1.

Hereinafter, the present invention will be described in more detail by way of examples. However, these examples are provided for illustrative purposes only and art not intended to limit the scope of the present invention.

**<Production Example: Production of Magnetic Particles>**

$Fe^{3+}$ (iron(III) chloride hexahydrate, 5.41 g) and $Fe^{2+}$ salt (iron(II) chloride tetrahydrate, 1.98 g) were added to 130 ml of distilled water and stirred at 400 to 600 rpm to prepare an aqueous magnetic particle solution.
Oleic acid (2 g) and 13 ml of acetone were mixed, and then added to the aqueous magnetic particle solution.
After stirring for 30 minutes, 15 ml of an aqueous ammonium hydroxide solution (solid content: 25 wt.%) was slowly added.
The temperature was raised to 80°C to 90°C and the mixture was stirred for 1 hour or more. Then, the temperature was lowered to 65°C to 75°C and 1 N HCl was added thereto until the pH of the reactant reached 2.
The mixture was washed with distilled water until the pH of the final reactant reached 7, and then dried at room temperature for at least 2 days to produce surface-treated magnetic particles (particle diameter: 500-600 nm, particle density: 5.15 g/cm$^3$). The diameter of the magnetic particles was measured using dynamic light scattering (DLS).
The true density of the magnetic particles was measured using a pycnometer.

**<Example: Preparation of microcarriers for cell culture>**

Example 1

(1) Preparation of dispersive phase

A mixture of styrene monomer (st), a crosslinking agent divinylbezene (DVB), the magnetic particles of Production Example and low-density oil (Isopar M, density 0.791 g/cm$^3$) in the content ratio shown in Table 1 below was stirred in 100 ml vial. Then, thermal initiator benzoyl peroxide (BPO, 2.1 wt.%) and tert-butyl peroxybenzoate (t-BP, 0.3 wt.%) were further added to a vial, and stirred at room temperature for 5 to 10 minutes. The content between each component is shown in Table 1 below.

(2) Preparation of continuous phase

6 g of PVA having a weight average molecular weight in the range of 85,000 to 125,000 and a hydrolysis rate of 87 to 89% was dissolved in 600 g of distilled water. Detailed contents are shown in Table 1 below.

(3) Production of particles by suspension polymerization

600 g of 1% PVA aqueous solution was mixed with the dispersive phase and stirred in an oil bath until a homogeneous dispersion was obtained. Specifically, the oil bath was gradually heated while stirring at 400 to 500 rpm at room temperature, and suspension polymerization was performed at a temperature of 80°C to 90°C and a speed of 400 to 600 rpm. The polymerization was performed under nitrogen purge.

(4) Obtaining particles

After 4 hours of reaction, the produced particles were recovered through a 45 μm sieve, washed 3 times with ethanol and 3 times with distilled water at 70 °C or more and 80°C or less, and dried in an oven at 80°C.

(5) Particle surface treatment

After polymerization, the particles dried at room temperature were immersed in tris buffer (pH 8.0) in which dopamine hydrochloride was dissolved at 1 mg/mL, stirred at room temperature for at least 1 hour to introduce the polydopamine layer onto the particle surface. After the reaction, particles were recovered through a 45 μm sieve, washed with ethanol three times, and dried in an oven at 80°C.

Examples 2 to 4

Particles were obtained through the same procedure as in Example 1, except that the composition of the dispersive phase and the continuous phase were adjusted as shown in Table 1 below.

Comparative Example 1

Particles were obtained through the same procedure as in Example 1, except that no magnetic particles were added.

Comparative Examples 2 and 3

Particles were obtained through the same procedure as in Example 1, except that the composition of the dispersive phase and the continuous phase were adjusted as shown in Table 1 below.

Reference Example 1

Particles were obtained through the same procedure as in Example 1, except that magnetic particles whose surface was not hydrophobically modified with oleic acid was added instead of the magnetic particles of Production Example.

[Table 1]

| | Magnetic particles (g) | | Low-density oil (g) | Styrene monomer (g) | Crosslinking agent (g) | Initiator (g) | Distilled water (g) |
|---|---|---|---|---|---|---|---|
| | Hydrophobic modification O (Production Example) | Hydrophobic modification X | | | | | |
| Example 1 | 0.01 | - | 25 | 70 | 25 | 1.96 | 600 |
| Example 2 | 0.1 | - | 25 | 70 | 25 | 1.96 | 600 |
| Example 3 | 0.5 | - | 25 | 70 | 25 | 1.96 | 600 |
| Example 4 | 1 | - | 25 | 70 | 25 | 1.96 | 600 |
| Comparative Example 1 | - | | 25 | 70 | 25 | 1.96 | 600 |
| Comparative Example 2 | 0.1 | - | - | 70 | 25 | 1.96 | 600 |
| Comparative Example 3 | 2 | - | - | 70 | 25 | 1.96 | 600 |
| Reference Example 1 | - | 2 | 25 | 70 | 25 | 1.96 | 600 |

**<Experimental Example: Measurement of physical properties of microcarrier for cell culture>**

The physical properties of the microcarriers for cell culture obtained in Examples, Comparative Examples and Reference Example were measured by the following methods, and the results are shown in Table 2 below.

**1. Particle size**

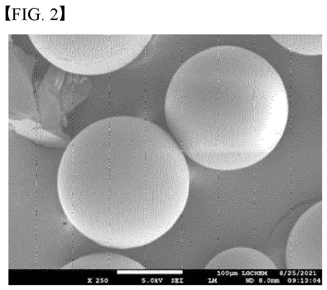

For the microcarriers for cell culture obtained in Examples, Comparative Examples and Reference Example, the diameters of 100 particles were measured through an optical microscope, and an arithmetic mean value thereof was obtained.
The SEM image of the microcarrier for cell culture obtained in Example 1 is shown in Fig. 2.

**2. Particle internal structure and pore diameter**

(1) Particle internal structure

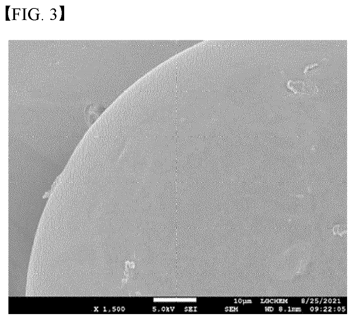

For the microcarriers for cell culture obtained in Example 1, the particle internal structure was confirmed through SEM. Specifically, after the particles was embedded in epoxy, a cross section was prepared through ion milling, and the cross-sectional shape of the particles was confirmed through SEM, which is shown in Fig. 3.

(2) Void diameter

After the microcarriers for cell culture obtained in Examples and Comparative Examples were embedded in epoxy, sections were prepared through ion milling, and the shape of the particle cross section was confirmed by SEM, and the minimum diameter and the maximum diameter among the pores inside the particles were obtained.

**3. Apparent Density**

For the microcarriers for cell culture obtained in Examples, Comparative Examples, and Reference Example, a sample in a condition in which the drying process has been completed prepared, and the sample was added to an aqueous ethanol solution having a density of 0.95 $g/cm^3$, 0.97 $g/cm^3$, 0.98 $g/cm^3$, or 0.995 $g/cm^3$ and distilled water having a density of 1 $g/cm^3$ (DIW) under conditions of room temperature (25°C) and atmospheric pressure (1 atm), respectively. Whether the particles floated or settled was confirmed, and the apparent density was evaluated under the following criteria.

    1) Floating in an aqueous ethanol solution with a density of 0.95 $g/cm^3$: less than 0.95 $g/cm^3$
    2) Settling in distilled water (DIW) having a density of 1 $g/cm^3$: greater than 1 $g/cm^3$
    3) Settling in an ethanol aqueous solution having a density of 0.95 g/cm3 and floating in distilled water (DIW) having a density of 1 $g/cm^3$: 0.95 $g/cm^3$ or more and less than 1 $g/cm^3$
    4) Settling in an aqueous ethanol solution having a density of 0.95 $g/cm^3$ and floating in an aqueous ethanol solution with a density of 0.97 $g/cm^3$: 0.95 $g/cm^3$ or more and less than 0.97 $g/cm^3$
    5) Settling in an aqueous ethanol solution having a density of 0.98 $g/cm^3$ and floating in an aqueous ethanol solution having a density of 0.995 $g/cm^3$: 0.98 $g/cm^3$ or more and less than 0.995 $g/cm^3$

**4. Specific Surface Area**

From the obtained apparent density, the specific surface area of the microcarrier for cell culture was calculated using the following Equation.

[Equation]

$$\text{Specific surface area of microcarrier } (cm^2/g) = 3 /(r*\rho)$$

in the above Equation, r means the average radius of the microcarriers, and $\rho$ means the apparent density of the microcarriers.

**5. Cell Culture Efficiency**

A culture medium containing mesenchymal stem cells (density: 1.05 $g/cm^3$) was filled in a 100mL bioreactor, to which 1 g to 1.5 g of the cell culture microcarriers obtained in Examples and Comparative Examples were added and stirred at 25 rpm. At this time, the temperature of the culture medium was maintained at 37°C, culture was continued for 5 days, and the cell culture efficiency of the microcarrier was evaluated according to the following criteria.

    Upper: Cell culture efficiency of 80% or more during cell culture
    Lower: Cell culture efficiency of less than 80% during cell culture

**6. Magnetic separation efficiency**

A strong magnet was brought into contact with one side of the vial containing the solution in which the microcarriers for cell culture obtained in Examples, Comparative Examples and Reference Example were dispersed, and it was visually confirmed whether the microcarriers for cell culture moved.

It was evaluated as "excellent" if 90% or more of the microcarriers for cell culture moved in the direction of the surface in contact with the magnet, and as "defective" if less than 90% of the microcarriers moved.

**7. Analysis of pore component**

With respect to the microcarriers for cell culture obtained in Examples, Comparative Examples and Reference Example, to analyze the components contained in the internal voids, the microcarriers for cell culture were freeze-pulverized and dissolved in chloroform, unreacted residual compounds were extracted, and detailed components were qualitatively and quantitatively analyzed through GC/FID (gas chromatography - flame ionization detector).

Specifically, standard samples of styrene (1/4000 mg/mL ~ 1 mg/mL), divinylbenzene (1/10000 mg/mL ~ 1 mg/mL), and Isopar M (1/400 mg/mL ~ 10 mg/mL) were prepared for each concentration in a mixed solvent of chloroform and methanol in a 1:2 volume ratio. 1 uL of the standard sample was injected into the GC/FID instrument and a calibration curve was prepared. 1 uL of the sample in Table 1 was injected and the content was calculated using the calibration curve. For GC/FID measurement, a column on Rtx (styrene, Isopar M) or wax (divinylbenzene) having an inner diameter of 0.53 mm, a length of 30 m, and a film thickness of 5 $\mu$m was used. Starting with an initial oven temperature of 50°C, the temperature was increased to 250°C at a rate of 10°C/min. As the mobile phase gas, helium gas at a rate of 15 mL/min was used.

At this time, the case in which oil or a component derived therefrom was detected is indicated by "O", and the case in which oil or a component derived therefrom was not detected is indicated by "X".

[Table 2]

| Experimental Example Measurement Results of Examples, Comparative Examples and Reference Example | | | | | | | |
|---|---|---|---|---|---|---|---|
| Category | Particle size ($\mu$m) | Void size ($\mu$m) | Apparent density (g/cm$^3$) | Specific surface area (cm$^2$/g) | Cell culture efficiency | Magnetic separation efficiency | Void component analysis |
| Example 1 | 168($\pm$24) | minimum: 1maximum:3 | 0.996 | 358.58 | Upper | Excellent | O |
| Example 2 | 159($\pm$21) | minimum:1 maximum:3 | 0.999 | 377.74 | Upper | Excellent | O |
| Example 3 | 154($\pm$32) | minimum: 1maximum:3 | 1.013 | 384.61 | Upper | Excellent | O |
| Example 4 | 164($\pm$35) | minimum: 1maximum:3 | 1.030 | 355.20 | Upper | Excellent | O |
| Comparative Example 1 | 179($\pm$36) | minimum: 1maximum: 3 | 0.995 | 336.88 | Upper | Defective | O |
| Comparative Example 2 | 174($\pm$24) | - | 1.054 | 327.16 | Lower | Excellent | X |
| Comparative Example 3 | 134($\pm$18) | - | 1.135 | 394.50 | Lower | Excellent | X |
| Reference Example 1 | 180($\pm$54) | minimum:1 maximum:3 | 1.064 | 323.62 | Upper | Defective | O |

As shown in Table 2, it could be confirmed that in the microcarriers for cell culture of Examples, the carrier culture is effectively performed under cell culture conditions while satisfying a low density of less than 1.05 g/cm$^3$, and

the magnetic separation efficiency is also excellent.

On the other hand, the microcarrier for cell culture of Comparative Example 1 does not contain magnetic particles, and thus has a problem that magnetic separation efficiency is lowered.

Further, in the microcarrier for cell culture of Comparative Example 2, the particle density is greater than 1.05 $g/cm^3$, and due to the density difference with the cells, smooth stirring is not performed in an incubator, resulting in a problem that cell adhesion is reduced.

Further, the microcarrier for cell culture of Comparative Example 3 has a particle density of greater than 1.05 $g/cm^3$, and due to the density difference with the cells, smooth stirring is not performed in the incubator, and cell adhesion is reduced, resulting in a problem that cell culture efficiency is low to such an extent that cell culture is impossible.

In addition, it could be confirmed that as the microcarrier for cell culture of Reference Example 1 contains magnetic particles that are not surface-treated with a hydrophobic functional group, the water-dispersed magnetic particles are not evenly dispersed in the dispersive phase containing a styrene monomer, and the water-dispersed magnetic particles agglomerate during polymerization. Further, in the case of the particles produced in this way, there was a problem that the particle size distribution was large and the magnetic separation efficiency was lowered.

**Claims**

1. A microcarrier for cell culture comprising: polystyrene-based particles that includes at least one of a hydrocarbon oil having 12 or more carbon atoms, and a void derived therefrom; and magnetic particles.

2. The microcarrier for cell culture according to claim 1 wherein:
   the magnetic particles include magnetic particles that are surface-treated with a hydrophobic functional group.

3. The microcarrier for cell culture according to claim 1 wherein:
   the magnetic particles include a hydrophobic ligand that contains a hydrophobic functional group on a surface of the magnetic particle, and the hydrophobic ligand includes at least one ligand selected from the group consisting of a fatty acid having 2 to 20 carbon atoms or a derivative thereof and a fatty acid amine having 2 to 20 carbon atoms or a derivative thereof.

4. The microcarrier for cell culture according to claim 1 wherein:
   the magnetic particles include at least one metal selected from the group consisting of gold (Au), silver (Ag), cobalt (Co), copper (Cu), iron (Fe), chromium (Cr), nickel (Ni), palladium (Pd), platinum (Pt) and tin (Sn) or an oxide thereof.

5. The microcarrier for cell culture according to claim 1 wherein:
   the magnetic particles exist in a dispersed state inside at least one of the hydrocarbon oil having 12 or more carbon atoms, and the void derived therefrom.

6. The microcarrier for cell culture according to claim 1 wherein:
   an apparent density of the microcarrier is 0.95 $g/cm^3$ or more and 1.05 $g/cm^3$ or less.

7. The microcarrier for cell culture according to claim 1 wherein:

   an average diameter of the microcarrier is 50 $\mu$m or more and 400 $\mu$m or less, and
   a specific surface area of the microcarrier is 200 $cm^2/g$ or more and 1000 $cm^2/g$ or less.

8. The microcarrier for cell culture according to claim 1 wherein:
   a diameter of the void is 0.1 $\mu$m or more and 5 $\mu$m or less.

9. The microcarrier for cell culture according to claim 1 wherein:
   a density of the hydrocarbon oil is 0.75 $g/cm^3$ or more and 0.80 $g/cm^3$ or less.

10. The microcarrier for cell culture according to claim 1 wherein:
    a density of the magnetic particles is 5 $g/cm^3$ or more and 6 $g/cm^3$ or less.

11. The microcarrier for cell culture according to claim 1 wherein:
    the hydrocarbon oil includes a linear or branched saturated hydrocarbon compound having 12 or more and 50 or less carbon atoms.

**12.** The microcarrier for cell culture according to claim 1 wherein:
the polystyrene-based particles include a suspension polymerization reaction product of a monomer composition containing a hydrocarbon oil having 12 or more carbon atoms, magnetic particles, and a styrene monomer.

**13.** The microcarrier for cell culture according to claim 12 wherein:
the monomer composition contains 0.01 parts by weight or more and 5 parts by weight or less of magnetic particles with respect to 100 parts by weight of the hydrocarbon oil having 12 or more carbon atoms.

**14.** The microcarrier for cell culture according to claim 12 wherein:
the monomer composition contains greater than 0.033 parts by weight and less than 3 parts by weight of an ethylenically unsaturated crosslinking agent with respect to 1 part by weight of the styrene monomer.

**15.** The microcarrier for cell culture according to claim 1 wherein:
the surface of the polystyrene-based particles further includes a primer polymer layer, a cell adhesion-inducing layer, or a combination layer thereof.

**16.** A method for producing a microcarrier for cell culture, which comprises subjecting a monomer composition containing magnetic particles and a styrene monomer to a suspension polymerization reaction in the presence of a hydrocarbon oil having 12 or more carbon atoms.

**17.** The method for producing a microcarrier for cell culture according to claim 16 wherein:
the magnetic particles include magnetic particles that are surface-treated with a hydrophobic functional group.

**18.** A cell culture composition comprising a cell and the microcarrier for cell culture of claim 1.

**19.** The cell culture composition according to claim 18 wherein:
the cell includes at least one compound selected from the group consisting of fibroblasts, chondrocyte, mesenchymal stem cells, CHO, HEK 293, vero cells, BHK21 and MDCK.

【FIG. 1】

【FIG. 2】

【FIG. 3】

【FIG. 4】

## INTERNATIONAL SEARCH REPORT

| International application No. |
| --- |
| **PCT/KR2022/014551** |

| A. | CLASSIFICATION OF SUBJECT MATTER |
| --- | --- |

**C12M 1/12**(2006.01)i; **C12N 11/14**(2006.01)i; **C12N 11/06**(2006.01)i; **C12N 11/08**(2006.01)i

According to International Patent Classification (IPC) or to both national classification and IPC

| B. | FIELDS SEARCHED |
| --- | --- |

Minimum documentation searched (classification system followed by classification symbols)

C12M 1/12(2006.01); B01F 13/08(2006.01); C08J 9/10(2006.01); C12M 3/00(2006.01); C12N 11/08(2006.01); C12N 13/00(2006.01); C12N 5/06(2006.01); H01F 1/147(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 마이크로 캐리어(micro carrier), 세포 배양(cell culture), 탄화수소 오일 (hydrocarbon oil), 폴리스티렌(polystyrene), 자성입자(magnetic particle), 소수성 작용기(hydrophobic functional group), 공극(pore)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | US 5863957 A (LI, N.-H. et al.) 26 January 1999 (1999-01-26)<br>See column 1, lines 27-31; column 5, line 8-column 6, line 56; column 8, line 23-column 9, line 19; column 19, line 52-column 20, line 6 and column 20, line 27-column 21, line 3. | 1-5,8-19 |
| Y | | 6-7 |
| Y | US 2016-0145600 A1 (CORNING INCORPORATED) 26 May 2016 (2016-05-26)<br>See claims 1-3 and 13-14; and paragraphs [0040]-[0042]. | 1-19 |
| Y | COSTA, F. T. et al. Highly magnetizable crosslinked chloromethylated polystyrene-based nanocomposite beads for selective molecular separation of 4-aminobenzoic acid. ACS Omega. 2019, vol. 4, pp. 5640-5649.<br>See abstract; pages 5641 and 5647; and figure 1. | 2-3,17 |
| Y | KR 10-2021-0011340 A (LG CHEM, LTD.) 01 February 2021 (2021-02-01)<br>See paragraphs [0022]-[0026] and [0048]-[0050]. | 6-7 |

☑ Further documents are listed in the continuation of Box C.    ☑ See patent family annex.

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **12 January 2023** | **13 January 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2022/014551** |

**C. DOCUMENTS CONSIDERED TO BE RELEVANT**

| Category* | Citation of document, with indication, where appropriate, of the relevant passages | Relevant to claim No. |
| --- | --- | --- |
| A | JP 2004-236553 A (HITACHI LTD. et al.) 26 August 2004 (2004-08-26)<br>See entire document. | 1-19 |
| A | JP 2004-313008 A (PENTAX CORPORATION) 11 November 2004 (2004-11-11)<br>See entire document. | 1-19 |

Form PCT/ISA/210 (second sheet) (July 2022)

**INTERNATIONAL SEARCH REPORT**
Information on patent family members

International application No.

**PCT/KR2022/014551**

| Patent document cited in search report | | | Publication date (day/month/year) | Patent family member(s) | | | Publication date (day/month/year) |
|---|---|---|---|---|---|---|---|
| US | 5863957 | A | 26 January 1999 | AU | 1995-26937 | B2 | 18 February 1999 |
| | | | | CN | 1150764 | A | 28 May 1997 |
| | | | | EP | 0764047 | A1 | 26 March 1997 |
| | | | | EP | 0764047 | B2 | 01 October 2008 |
| | | | | JP | 10-501173 | A | 03 February 1998 |
| | | | | JP | 2009-057578 | A | 19 March 2009 |
| | | | | US | 5583162 | A | 10 December 1996 |
| | | | | US | 5653922 | A | 05 August 1997 |
| | | | | US | 5760097 | A | 02 June 1998 |
| | | | | US | 6100306 | A | 08 August 2000 |
| | | | | WO | 95-33553 | A1 | 14 December 1995 |
| US | 2016-0145600 | A1 | 26 May 2016 | None | | | |
| KR | 10-2021-0011340 | A | 01 February 2021 | EP | 3889252 | A1 | 06 October 2021 |
| | | | | EP | 3889252 | A4 | 15 June 2022 |
| | | | | JP | 2022-521518 | A | 08 April 2022 |
| | | | | KR | 10-2467863 | B1 | 16 November 2022 |
| | | | | US | 2022-0081684 | A1 | 17 March 2022 |
| | | | | WO | 2021-015547 | A1 | 28 January 2021 |
| JP | 2004-236553 | A | 26 August 2004 | JP | 2005-236553 | A5 | 15 December 2005 |
| JP | 2004-313008 | A | 11 November 2004 | FR | 2853664 | A1 | 15 October 2004 |
| | | | | GB | 2400378 | A | 13 October 2004 |
| | | | | GB | 2400378 | B | 21 November 2007 |
| | | | | GB | 2413564 | A | 02 November 2005 |
| | | | | GB | 2413564 | B | 10 May 2006 |
| | | | | JP | 2004-313007 | A | 11 November 2004 |
| | | | | US | 2004-0248291 | A1 | 09 December 2004 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020210128333

- KR 1020220112921